(19) Europäisches Patentamt
European Patent Office
Office européen des brevets

(11) Veröffentlichungsnummer: **0 477 901 A2**

(12) **EUROPÄISCHE PATENTANMELDUNG**

(21) Anmeldenummer: **91116334.3**

(22) Anmeldetag: **25.09.91**

(51) Int. Cl.[5]: **C07D 239/32**, C07D 239/26, C09K 19/34

(30) Priorität: **27.09.90 DE 4030579**

(43) Veröffentlichungstag der Anmeldung: **01.04.92 Patentblatt 92/14**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Illian, Gerd, Dr.**
**Rauenthaler Weg 32**
**W-6000 Frankfurt am Main(DE)**
Erfinder: **Wingen, Rainer, Dr.**
**Brunnenstrasse 1**
**W-6234 Hattersheim am Main(DE)**
Erfinder: **Müller, Ingrid, Dr.**
**Rautenweg 1**
**W-6272 Niedernhausen(DE)**

(54) **Cyclohexylphenylpyrimidine, Verfahren zu ihrer Herstellung und ihre Verwendung in flüssigkristallinen Mischungen.**

(57) Cyclohexylphenylpyrimidine der allgemeinen Formel (I)

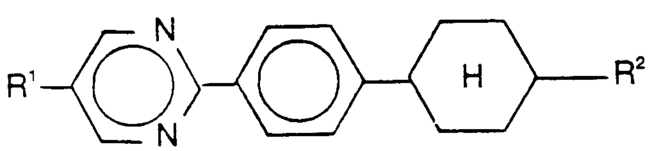

(I)

in der bedeuten:

$R^1$ geradkettiges oder verzweigtes, chirales oder achirales Alkyl oder Alkenyl mit 2 bis 16 C-Atomen, wobei eine oder zwei nicht benachbarte $-CH_2$-Gruppen durch -O-, -S-, -CO-, -COO-, -OCO-, $-Si(CH_3)_2-$ oder $-C(CH_3)_2$-ersetzt sein können, und wobei auch ein oder mehrere H-Atome des Alkyl bzw. Alkenylrestes durch F-Atome ersetzt sein können, und wobei die endständige $CH_3$-Gruppe des Alkyls auch durch

$-CH_2-$ 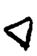

ersetzt sein kann,

$R^2$ geradkettiges oder verzweigtes, chirales oder achirales Alkyl mit 1 bis 10 C-Atomen, sind besonders geeignete Komponenten für ferroelektrische Flüssigkristallmischungen, da sie beim Einsatz in elektrooptischen Schalt- und Anzeigeelementen zu günstigen Eigenschaften wie z.B. hohem Kontrastführen.

EP 0 477 901 A2

Die ungewönliche Kombination von anisotropem und fluidem Verhalten der Flüssigkristalle hat zu ihrer Verwendung in elektro-optischen Schalt- und Anzeigevorrichtungen geführt. Dabei können ihre elektrischen, magnetischen, elastischen und/oder thermischen Eigenschaften zu Orientierungsänderungen genutzt werden. Optische Effekte lassen sich beispielsweise mit Hilfe der Doppelbrechung, der Einlagerung dichroitisch absorbierender Farbstoffmoleküle ("guest-host mode") oder der Lichtstreuung erzielen.

Insbesondere haben ferroelektrische Flüssigkristalle in jüngerer Zeit Interesse als Anzeigemedium in elektro-optischen Bauelementen (Displays) gewonnen (z.B. Lagerwall et al. "Ferroelectric Liquid Crystals for Displays", SID Symposium, October Meeting 1985, San Diego, Ca. USA).

Für die praktische Verwendung von ferroelektrischen Flüssigkristallen in elektrooptischen Anzeigen werden chirale, geneigt-smektische Phasen wie $S_c$-Phasen benötigt [R.B. Meyer, L. Liebert, L. Strzelecki und P. Keller, J. Physique 36, L-69 (1975)], die über einen großen Temperaturbereich stabil sind. Diese Ziel kann man erreichen mit Verbindungen, die selbst solche Phasen, z. B. $S_c^*$-Phasen, ausbilden, oder aber, indem man nicht chirale, geneigt-smektische Phasen ausbildende Verbindungen mit optisch aktiven Verbindungen dotiert [M. Brunet, Cl. Williams, Ann. Phys. 3,237 (1978)].

Weiterhin ist bei der Verwendung ferroelektrischer Flüssigkristallmischungen in elektro-optischen Bauelementen eine einheitliche planare Orientierung der Flüssigkristalle notwendig, um ein hohes Kontrastverhältnis zu erzielen. Es hat sich gezeigt, daß sich eine einheitliche planare Orientierung in der $S_c$-Phase erreichen läßt, wenn die Phasenfolge der Flüssigkristallmischung mit abnehmender Temperatur lautet: Isotrop → nematisch → smektisch A → smektisch C (siehe z.B. K. Flatischler et al., Mol. Cryst. Liq. Cryst. 131, 21 (1985); T. Matsumoto et al., P. 468-470, Proc. of the 6th Int. Display Research Conf., Japan Display, 30. September-2. October 1986, Tokyo, Japan; M. Murakami et al., ibid. p. 344-347).

Für ferroelektrische (chiral smektische) Flüssigkristallmischungen muß zusätzlich die Bedingung erfüllt sein, daß die Ganghöhe (pitch) der Helix in der $S_c^*$-Phase groß, d.h. größer als 5 $\mu$m, und in der $N^*$-Phase sehr groß, d.h. größer als 10 $\mu$m bzw. unendlich ist.

Die optische Schaltzeit $\tau[\mu s]$ ferroelektrischer Flüssigkristallsysteme, die möglichst kurz sein soll, hängt von der Rotationsviskosität des Systems $\gamma$[mPas], der spontanen Polerisation $P_s[nC/cm^2]$ und der elektrischen Feldstärke E[V/m] ab nach der Beziehung

$$\tau \approx \frac{\gamma}{Ps.E}$$

Da die Feldstärke E durch den Elektrodenabstand im elektrooptischen Beuteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$, vorzugsweise <0.13, und eine geringe positive oder vorzugsweise negative dielektrische Anisotropie $\Delta\epsilon$ verlangt. (siehe S.T. Legerwall et al., "Ferroelectric Liquid Crystals for Displays" SID Symposium, Oct. Meeting 1985, Sen Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_c$ aufweisen. Weitere Komponenten der Mischung werden oftmals zur Schmelzpunktserniedrigung und zur Verbreiterung der $S_c$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll. Hierbei zeigt sich, daß die Verwendung von Mischungskomponenten, die nur zwei aromatische Kerne enthalten, zu einer niedrigen Viskosität der Mischung führt.

Einzelne dieser Komponenten und auch bestimmte Mischungen sind bereits aus dem Stand der Technik bekannt. Da aber die Entwicklung, insbesondere von ferroelektrischen Flüssigkristallmischungen, noch in keiner Weise als abgeschlossen betrachtet werden kann, sind die Hersteller von Displays an unterschiedlichen Mischungen interessiert. Dieses u.a. auch deshalb, weil erst das Zusammenwirken der flüssigkristallinen Mischungen mit den einzelnen Bauteilen der Anzeigevorrichtungen bzw. der Zellen (z.B. der Orientierungsschicht) Rückschlüsse auf die Qualität auch der flüssigkristallinen Mischungen zuläßt.

Es ist bekannt, daß bestimmte Derivate des Phenylpyrimidins, insbesondere 5-Alkyl-2-(4-alkyloxyphenyl)-pyrimidine, $S_c$-, $S_A$- und N-Phasen ausbilden können (siehe D. Demus und H. Zaschke, "Flüssige Kristalle in Tabellen", VEB Deutscher Verlag für Grundstoffindustrie, Leipzig 1974, S. 260-261) und daneben

durch Zusatz optisch aktiver Dotierstoffe in ferroelektrische Flüssigkristallmischungen umgewandelt werden können [siehe L.M. Blinov et al., Sow. Phys. Usp. 27 (7), 492 (1984); L.A. Beresnew et al., Ferroelectrics, 59 [321]/1 (1984), vorgetragen auf der 5th. Conference of Soc. Countries on Liquid Crystals, Odessa, UdSSR, Oct. 1983; DE-A 35 15 347, EP-A 0 206 228, EP-A 0 225 195].

Es ist weiter bekannt, daß tiefere Schmelzpunkte und eine Verbreiterung der gewünschten flüssigkristallinen Phasen durch Mischen mehrerer flüssigkristalliner Verbindungen erreicht werden [siehe D. Demus et al., Mol. Cryst. Liq. Cryst. 25, 215 (1974), J.W. Goodby, Ferroelectrics 49, 275 (1983)], und daß die Schmelzpunktsdepression umso ausgeprägter ist, je stärker sich auch die Mischungskomponenten strukturell unterscheiden (z.B. J.S. Dave et al., J. Chem. Soc. 1955, 4305).
Dies gilt auch für die Schmelzpunktsdepression in Systemen, die eine für die Herstellung elektrooptischer Bauelemente ideale Phasenfolge $X \leftrightarrow S_c \leftrightarrow S_A \leftrightarrow N \leftrightarrow I$ aufweisen. Hier bleiben aber andere wesentliche Kenngrößen eher dann erhatten, wenn die Komponenten der Mischung strukturell ähnlich sind und selber diese Phasenfolge aufweisen. Die beiden Aufgaben - Schmelzpunktserniedrigung und Verschiebung der unteren Temperaturgrenze der $S_c$-Phase zu tieferer Temperatur einerseits und weitgehende Erhaltung der anderen Kenngrößen andererseits - stehen also in einem Widerspruch.

Es wurde bereits vorgestellt, daß Flüssigkristalle mit einer endständigen Cyclopropylgruppe (siehe DE-A 3 915 804 und DE-A 3839 330) sowie Flüssigkristalle mit einer geminalen Dimethyl-substitution (siehe DE-A 4 003 012) bzw. mit einer Dimethylsilyl-substitution (siehe DE-A 3 827 600) in der Kette zur Verwendung in flüssigkristallinen Mischungen geeignet sind.

Die Dimethyl-verzweigten Verbindungen weisen aber vergleichsweise schmale nematische Phasenbereiche auf. Das gilt insbesondere für Verbindungen mit nur zwei aromatischen Kernen, die aufgrund ihrer niedrigen Viskosität gegenüber Verbindungen mit drei aromatischen Kernen bevorzugt sind.
Die in DE-A 4 003 012 und DE-A 3 827 600 vorgestellten Verbindungen mit zwei aromatischen Kernen weisen jedoch entweder keine nematische oder keine $S_A$-Phase und/oder relativ hohe Schmelzpunkte und unbefriedigende Schmelzpunktseigenschaften in Mischungen auf.

Auch bei den in DE-A 3 915 804 vorgestellten Substanzen mit zwei aromatischen Ringen weisen die Beispiele mit den höchsten Klärpunkten keine $S_A$-Phase auf. Die Verwendung dieser Komponenten in Mischung führt dann zwar oft zu einer Erhöhung der Temperatur der nematischen und der $S_c$-Phase, aber auch zu einem Verschwinden der $S_A$-Phase.

Es wurde nun gefunden, daß spezielle dreikernige Verbindungen die eingangs gestellte Aufgabe - Gewährleistung von niedrigem Schmelzpunkt und breiten flüssigkristallinen Phasen bei gleichzeitiger Beibehaltung günstiger elektropischer Eigenschaften in Mischungen (Schaltzeiten, Viskositäten, Schaltwinkel) - lösen. Die Erfindung betrifft ein Cyclohexylphenylpyrimidin der allgemeinen Formel (I)

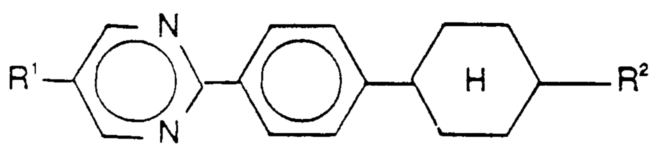

(I)

in der bedeuten:

$R^1$ geradkettiges oder verzweigtes, chirales oder achirales Alkyl oder Alkenyl mit 2 bis 16 C-Atomen, wobei eine oder zwei nicht benachbarte $-CH_2$-Gruppen durch -O-,-S-,-CO-, -COO-, -OCO-, $-Si(CH_3)_2-$ oder $-C(CH_3)_2-$ersetzt sein können, und wobei auch ein oder mehrere H-Atome des Alkyl- bzw. Alkenylrestes durch F-Atome ersetzt sein können, und wobei die endständige $CH_3$-Gruppe des Alkyls auch durch

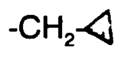

ersetzt sein kann,

$R^2$ geradkettiges oder verzweigtes, chirales oder achirales Alkyl mit 1 bis 10 C-Atomen.

Bevorzugt wird ein Cyclohexylphenylpyrimidin der oben genannten Formel (I), bei dem

$R^1$ ein geradkettiges oder verzweigtes, chirales oder achirales Alkyl mit 2 bis 16 C-Atomen ist, bei dem eine $-CH_2$-Gruppe durch $-C(CH_3)_2-$ oder $-Si(CH_3)_2-$ersetzt ist und bei dem eine dazu nicht benachbarte $-CH_2$-Gruppe durch -O-, -S-, -COO- oder -OCO- ersetzt sein kann.

Ebenfalls bevorzugt wird ein Cyclohexylphenylpyrimidin, bei dem

$R^1$ ein geradkettiges oder verzweigtes, chirales oder achirales Alkyl mit 2 bis 16 C-Atomen ist, bei dem die entständige $CH_3$-Gruppe durch

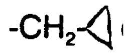

ersetzt ist, und bei dem eine $-CH_2$-Gruppe durch -O, -S-, -COO- oder -O-CO-ersetzt sein kann.

Auch bevorzugt bedeutet $R^1$ ein geradkettiges Alkyl mit 4 bis 12 C-Atomen, wobei eine $-CH_2$-Gruppe durch -O- oder -S- ersetzt sein kann.

In einer weiteren Ausführungsform der Erfindung ist

$R^1$ ein geradkettiges Alkyl mit 4 bis 12 C-Atomen, bei dem eine $-CH_2$-Gruppe durch $-C(CH_3)_2$- ersetzt ist und eine weitere, dazu nicht benachbarte $-CH_2$-Gruppe durch -O- ersetzt sein kann, oder

$R^1$ bedeutet ein geradkettiges Alkyl mit 4 bis 12 C-Atomen, bei dem die endständige $CH_3$-Gruppe durch

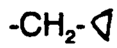

substituiert ist.

$R^2$ bedeutet vorzugsweise ein geradkettiges Alkyl mit 2 bis 8 C-Atomen.

Eine weitere Lösung der gestellten Aufgabe ist eine flüssigkristalline, insbesondere eine ferroelektrische flüssigkristalline Mischung, die mindestens eine Verbindung der allgemeinen Formel (I) enthält.

Die Flüssigkristallmischungen bestehen im allgemeinen aus mindestens 2, vorzugsweise 2 bis 20, insbesondere bevorzugt 2 bis 15 Komponenten, darunter mindestens eine der erfindungsgemäß beanspruchten Verbindungen. Die anderen Bestandteile werden vorzugsweise ausgewählt aus den bekannten Verbindungen mit nematischen, cholesterischen und/oder geneigt-smektischen Phasen, dazu gehören beispielsweise Schiff'sche Basen, Biphenyle, Terphenyle, Phenylcyclohexane, Cyclohexylbiphenyle, Pyrimidine, Zimtsäureester, Cholesterinester sowie verschiedene überbrückte, terminal-polare mehrkernige Ester von p-Alkylbenzoesäuren. Im allgemeinen liegen die im Handel erhältlichen Flüssigkristallmischungen bereits vor der Zugabe der erfindungsgemäßen Verbindung(en) als Gemische verschiedener Komponenten vor, von denen mindestens eine mesogen ist, d.h. als Verbindung, in derivatisierter Form oder im Gemisch mit bestimmten Komponenten eine Flüssigkristall-Phase zeigt [mindestens eine enantiotrope (Klärtemperatur < Schmelztemperatur) oder monotrope (Klärtemperatur > Schmelztemperatur) Mesophasenbildung erwarten läßt].

Von dem oder den erfindungsgemäßen Cyclohexylphenylpyrimidin(en) enthalten die Flüssigkristallmischungen im allgemeinen 0,1 bis 70 Mol-%, bevorzugt 0,5 bis 50 Mol-%, insbesondere 1 bis 25 Mol-%.

Bevorzugt sind Mischungen, die neben mindestens einer erfindungsgemäßen Verbindung 5-Alkyloxy-2-(4-alkyloxyphenyl)-pyrimidine, 5-Alkyl-2-(4-alkyloxyphenyl)-pyrimidine, bzw. entsprechende Phenylpyridine enthalten, bei denen ein Alkylrest durch ein endständiges Cyclopropyl substituiert sein kann.

Für die gebrauchsfertigen ferroelektrischen Flüssigkristallmischungen wurden die Werte für die spontane Polarisation $P_s$[nC/cm$^2$], den Kontrast K und die optische Schaltzeit $\tau$[$\mu$s], wobei alle Messungen bei einer Temperatur von 25 °C vorgenommen wurden, gemessen.

Die $P_s$-Werte werden nach der Methode von H. Diamant et al. (Rev. Sci. Instr., 28, 30, 1957) gemessen, wobei Meßzellen mit 2 $\mu$m Elektrodenabstand und geriebenem Polyimid als Orientierungsschicht verwendet werden.

Zur Bestimmung von $\tau$ und K wird die Meßzelle auf dem Drehtisch eines Polarisationsmikroskops zwischen gekreuztem Analysator und Polarisator befestigt. Für die Bestimmung des Kontrastes (K) wird die Meßzelle durch Drehen so positioniert, daß eine Photodiode minimalen Lichtdurchgang anzeigt (Dunkelzustand). Die Mikroskopbeleuchtung wird so geregelt, daß die Photodiode für alle Zellen die gleiche Lichtintensität anzeigt. Nach einem Schaltvorgang ändert sich die Lichtintensität (Hellzustand) und der Kontrast wird aus dem Verhältnis der Lichtintensitäten dieser Zustände berechnet.

Mit Hilfe einer Photodiode erfolgt die Bestimmung der Schaltzeit $\tau$, indem die Anstiegszeit des Lichtsignals von 10 auf 90 % Signalhöhe gemessen wird. Die Schaltspannung besteht aus Rechteckpulsen und beträgt ± 10 V/$\mu$m.

Die Phasenumwandlungstemperaturen werden beim Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen

Nematisch (N bzw. N*)

Smektisch-C ($S_c$ bzw. $S_c$*)

Smektisch-A ($S_A$ bzw. $S_A^*$)
Kristallin (X)
erfolgt in ° C und die Werte stehen zwischen den Phasenbezeichnungen in der Phasenfolge.

Die erfindungsgemäßen Mischungen lassen sich vorteilhaft als Komponente in ferroelektrischen Flüssigkristall-Schalt- und -Anzeigevorrichtungen einsetzen. Diese Vorrichtungen enthalten im allgemeinen Trägerplatten, Elektroden, mindestens einen Polarisator, Orientierungsschichten sowie ein flüssigkristallines Medium. Im einzelnen wird auf die eingangs genannte Literaturstelle Bezug genommen.

Die Herstellung der erfindungsgemäßen Verbindungen kann nach an sich literaturbekannten Verfahren erfolgen. So können z.B. Ringschlußreaktionen, wie beschrieben bei Zaschke et al., J. Prakt. Chem. 312, 494 (1970), zum Aufbau der Pyrimidineinheit angewendet werden (Schemata 1 und 2).

Schema 1

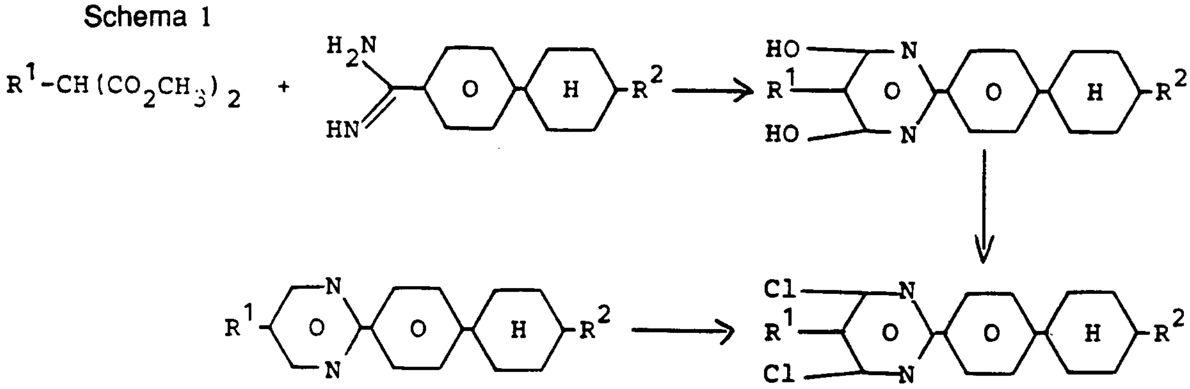

Schema 2

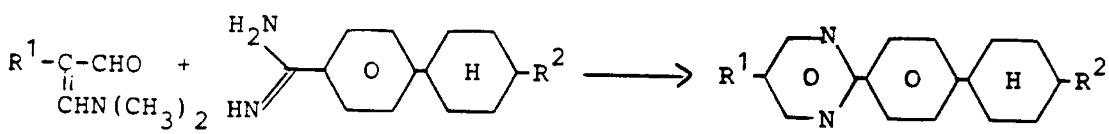

Es kann auch ein nach Schema 1 oder 2 aufgebautes Pyrimidinderivat mit phenylständigem Halogensubstituenten über metallorganische Zwischenstufen mit einem Cyclohexan-Fragment verknüpft werden.

Schema 3

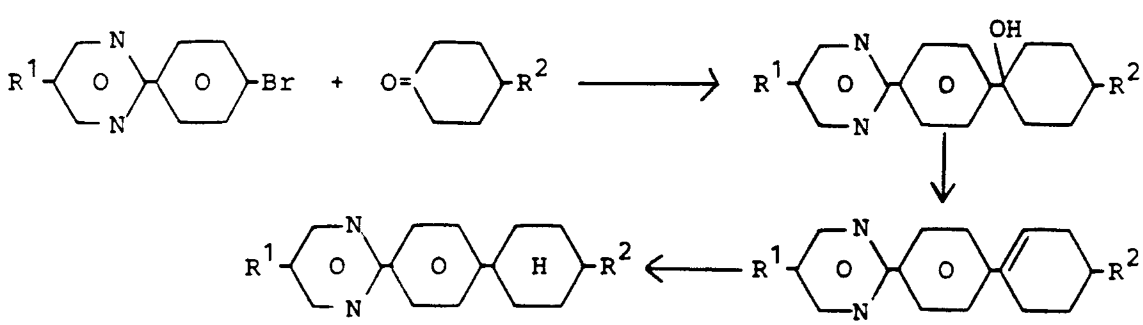

Schließlich ist es auch möglich, die metallorganische Kopplung gemäß Schema 4 zwischen einem Pyrimidin- und einem Cyclohexyl-Fragment vorzunehmen.

## Schema 4

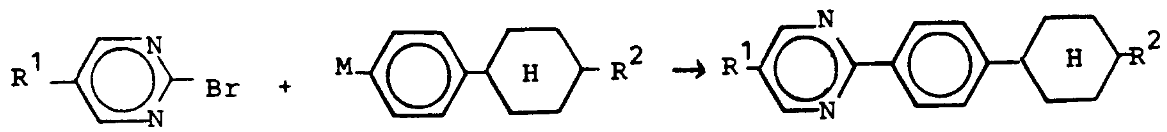

Die beschriebenen Flüssigkristallmischungen lassen sich vorteilhaft in elektrooptischen Schalt- und Anzeigevorrichtungen (FLC-Lichtventilen bzw. Displays) einsetzen. Diese weisen u.a. folgende Bestandteile auf: eine erfindungsgemäße flüssigkristalline Mischung (enthaltend ein Cyclohexylphenylpyrimidin), Trägerplatten (z.B. aus Glas oder Kunststoff), beschichtet mit transparenten Elektroden (zwei Elektroden), mindestens eine Orientierungsschicht, Abstandshalter, Kleberahmen, Polarisatoren sowie für Farbdisplays dünne Farbfilterschichten. Weitere mögliche Komponenten sind Antireflex-, Passivierungs-, Ausgleichs- und Sperrschichten sowie elektrisch nichtlineare Elemente, wie z.B. Dünnschichttransistoren (TFT) und Metall-Isolator-Metall-(MIM)-Elemente. Im Detail ist der allgemeine Aufbau von Flüssigkristalldisplays bereits in einschlägigen Monographien beschrieben (z.B. E. Kaneko, "Liquid Crystal TV Displays: Principles and Applications of Liquid Crystal Displays", KTK Scientific Publishers, 1987, Seiten 12-30 und 163-172).

Die Erfindung wird durch die nachfolgenden Beispiele näher erläutert:

Beispiel 1

Synthese von 5-(8-Cyclopropyloctyloxy)-2-(4"-trans-pentyl-cyclohexyl-4-phenyl)pyrimidin

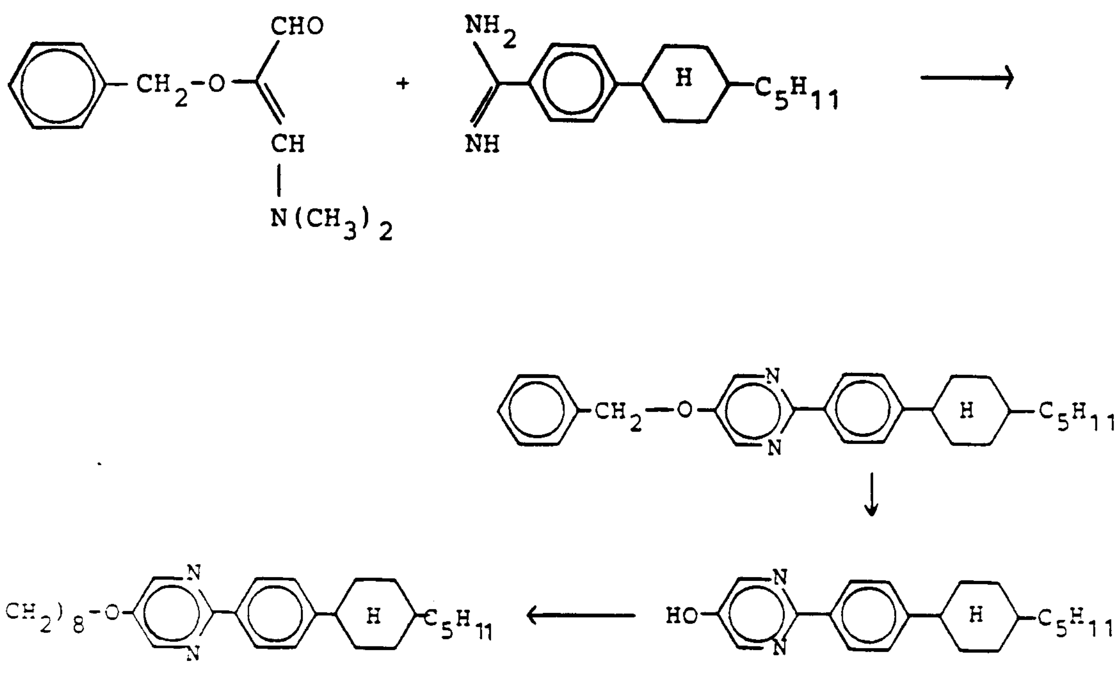

Eine Lösung von 1.94 g 5-Hydroxy-2-[4-(4-trans-pentylcyclohexyl)phenyl]pyrimidin-erhalten durch Kondensation von 2-Benzyloxy-3-dimethyl-amino-acrolein mit 4-(4-trans-Pentylcyclohexyl)benzamidin und nachfolgender Abspaltung ($H_2$/Pd/THF) der Benzylschutzgruppe des primär gebildeten 5-Benzyloxy-2-[4-(4-trans-pentylcyclohexyl)phenyl]pyrimidins - in 50 ml Dimethylformamid wird mit 0.36 g Natriumhydrid (60 %) versetzt. Nach Abklingen der Reaktion werden 2.1 g 8-Cyclopropyloctylbromid zugegeben und 3 h bei einer Temperatur von 60 ° C gerührt. Nach Hydrolyse und Extraktion mit Dichlormethan werden 3.2 g Rohprodukt erhalten; Reinigung erfolgt durch Chromatographie ($SiO_2/CH_2Cl_2$) und Umkristallisation aus n-Hexan.

Anwendungsbeispiel 1

a) Eine Mischung besteht aus den Komponenten

| | |
|---|---|
| 5-Octyloxy-2-(4-hexyloxy-phenyl)-pyrimidin | 23.8 Mol-% |
| 5-Octyloxy-2-(4-octyloxy-phenyl)-pyrimidin | 11 Mol-% |
| 5-Octyloxy-2-(4-butyloxy-phenyl)-pyrimidin | 25.2 Mol-% |
| 5-Octyloxy-2-(4-decyloxy-phenyl)-pyrimidin | 20 Mol-% |
| 5-(8-Cyclopropyloloxy)-2-(4"-trans-propylcyclohexyl-4'-phenyl)-pyrimidin | 20 Mol-% |

und zeigt folgende flüssigkristalline Phasenbereiche:

X 11 $S_c$ 85 $S_A$ 99.5 N 109 I

b) Im Vergleich dazu weist die in DE-A 38 31 226 beschriebene flüssigkristalline Mischung, die sich von der obengenannten Mischung nur dadurch unterscheidet, daß sie keine Cyclohexylphenylpyrimidinkomponente enthält, folgende Phasenbereiche auf:

X 13 $S_C$ 81 $S_A$ 95 N 98 I

Die Zugabe der erfindungsgemäßen Verbindung führt zu einer Erniedigung des Schmelzpunktes und einer Erhöhung der flüssigkristallinen Phasenbereiche.

Anwendungsbeispiel 2

a) Eine Mischung besteht aus den Komponenten

| | |
|---|---|
| 5-Octyloxy-2-(4-hexyloxy-phenyl)-pyrimidin | 23.8 Mol-% |
| 5-Octyloxy-2-(4-octyloxy-phenyl)-pyrimidin | 11 Mol-% |
| 5-Octyloxy-2-(4-butyloxy-phenyl)-pyrimidin | 25.2 Mol-% |
| 5-Octyloxy-2-(4-decyloxy-phenyl)-pyrimidin | 20 Mol-% |
| 5-(8-Cyclopropyloctyloxy)-2-(4''-trans-pentylcyclohexyl-4'-phenyl)-pyrimidin | 20 Mol-% |

und zeigt folgende flüssigkristalline Phasenbereiche:

X 11 $S_C$ 83.5 $S_A$ 100 N 110 I

b) Im Vergleich dazu weist eine in DE-A 38 31 226 beschriebene flüssigkristalline Mischung, die sich von der obengenannten Mischung nur dadurch unterscheidet, daß sie keine Cyclohexylphenylpyrimidinkomponente enthält, folgende Phasenbereiche auf:

X 13 $S_c$ 81 $S_A$ 95 N 98 I

Die Zugabe der erfindungsgemäßen Verbindung führt zu einer Erniedigung des Schmelzpunktes und einer Erhöhung der flüssigkristallinen Phasenbereiche.

Anwendungsbeispiel 3

a) Eine Mischung aus den Komponenten

| | |
|---|---|
| 5-Octyloxy-2-(4-hexyloxy-phenyl)-pyrimidin | 23.8 Mol-% |
| 5-Octyloxy-2-(4-octyloxy-phenyl)-pyrimidin | 11 Mol-% |
| 5-Octyloxy-2-(4-butyloxy-phenyl)-pyrimidin | 25.2 Mol-% |
| 5-Octyloxy-2-(4-decyloxy-phenyl)-pyrimidin | 20 Mol-% |
| 5-(8-Cyclopropyloctyloxy)-2-(4''-trans-propylcyclohexyl-4'phenyl)-pyrimidin | 16 Mol-% |
| 5-(7,7-Dimethyl-undecyloxy)-2-(4''-trans-propylcyclohexyl-4'phenyl)-pyrimidin | 4 Mol-% |

zeigt folgende flüssigkristalline Phasenbereiche:

X 11 $S_C$ 84 $S_A$ 97 N 108 I

b) Im Vergleich dazu weist die in DE-A 38 31 226 beschriebene flüssigkristalline Mischung, die sich von der obengenannten Mischung nur dadurch unterscheidet, daß sie keine Cylohexylphenylpyrimidinkomponenten enthält, folgende Phasenbereiche auf:

X 13 $S_C$ 81 $S_A$ 95 N 98 I

Die Zugabe der erfindungsgemäßen Verbindungen führt zu einer Erniedigung des Schmelzpunktes und einer Erhöhung der flüssigkristallinen Phasenbereiche.

Anwendungsbeispiel 4

a) Eine Mischung aus den Komponenten

| | |
|---|---|
| 5-Octyloxy-2-(4-hexyloxy-phenyl)-pyrimidin | 23.8 Mol-% |
| 5-Octyloxy-2-(4-octyloxy-phenyl)-pyrimidin | 11 Mol-% |
| 5-Octyloxy-2-(4-butyloxy-phenyl)-pyrimidin | 25.2 Mol-% |
| 5-Octyloxy-2-(4-decyloxy-phenyl)-pyrimidin | 20 Mol-% |
| 5-[4-(Butyl-dimethylsilyl)butyloxy]-2-(4''-trans-pentylcyclohexyl-4'phenyl)-pyrimidin | 20 Mol-% |

zeigt folgende flüssigkristalline Phasenbereiche:

X 9 $S_C$ 77 $S_A$ 81 N 97 I

b) Im Vergleich dazu weist die in DE-A 38 31 226 beschriebene flüssigkristalline Mischung, die sich von der obengenannten Mischung nur dadurch unterscheidet, daß sie keine Cyclohexylphenylpyrimidinkomponente enthält, folgende Phasenbereiche auf:

X 13 $S_C$ 81 $S_A$ 95 N 98 I

Die Zugabe der erfindungsgemäßen Verbindung führt zu einer Erniedigung des Schmelzpunktes und einer Erhöhung der flüssigkristallinen Phasenbereiche.

Anwendungsbeispiel 5

a) Eine Mischung aus den Komponenten

| | |
|---|---|
| 5-Octyl-2-(4-hexyloxy-phenyl)-pyrimidin | 20,5 Mol-% |
| 5-Octyl-2-(4-octyloxy-phenyl)-pyrimidin | 13,6 Mol-% |
| 5-Octyl-2-(4-decyloxy-phenyl)-pyrimidin | 13,3 Mol-% |
| 5-Octyl-2-(4'-(6''-cyclopropylhexyloxy)-phenyl)-pyridin | 16,6 Mol-% |
| 5-Octyl-2-(4'-(6''-cyclopropylhexyl-carbonyloxy-phenyl) -pyrimidin | 16,0 Mol-% |
| 5-(8-Cyclopropyloctyloxy)-2-(4''-trans-propylcyclohexyl -4'phenyl)-pyrimidin | 20,0 Mol-% |

zeigt folgende flüssigkristalline Phasenbereiche:

X 10 $S_C$ 64 $S_A$ 73 N 82 I

b) Im Vergleich dazu weist die flüssigkristalline Mischung, die sich von der obengenannten Mischung nur dadurch unterscheidet, daß sie keine Cyclohexylphenylpyrimidinkomponente enthält, folgende Phasenbereiche auf.

X -3 $S_C$ 50 $S_A$ 60 N 62 I

Die Zugabe der erfindungsgemäßen Verbindung führt zu einer Erniedigung des Schmelzpunktes und einer Verbreiterung der flüssigkristallinen Phasenbereiche. Dieses Beispiel belegt, daß die erfindungsgemäßen Verbindungen auch in Mischungen geeignet sind, die neben Phenylpyrimidinen auch Phenylpyridine enthalten.

Anwendungsbeispiel 6

a) Eine Mischung aus den Komponenten

| | |
|---|---|
| 5-Octyloxy-2-(4-hexyloxy-phenyl)-pyrimidin | 11,4 Mol-% |
| 5-Octyloxy-2-(4-butyloxy-phenyl)-pyrimidin | 12,6 Mol-% |
| 5-Octyloxy-2-(4-decyloxy-phenyl)-pyrimidin | 6,9 Mol-% |
| 5-Octyloxy-2-(4-octyloxy-phenyl)-pyrimidin | 3,8 Mol-% |
| 5-Octyloxy-2-(4-dodecyloxy-phenyl)-pyrimidin | 6,9 Mol-% |
| 5-Octyl-2-(4-dodecyloxy-phenyl)-pyrimidin | 10,2 Mol-% |
| trans-4-Pentyl-cyclohexancarbonsäure-(4'-(5-decylpyrimidin-2-yl))-phenylester | 16,2 Mol-% |
| Heptansäure-(4-(5-octylpyrimidin-2-yl)) phenylester | 17,0 Mol-% |
| 5-(8-Cyclopropyloctyloxy)-2-(4''-trans-propyl-cyclohexyl-4'-phenyl)-pyrimidin | 15,0 Mol-% |

zeigt folgende flüssigkristalline Phasenbereiche:

X -7 $S_C$ 78 $S_A$ 85 N 105 I

b) Im Vergleich dazu weist die in DE-A 38 31 226 beschriebene flüssigkristalline Mischung, die sich von der obengenannten Mischung nur dadurch unterscheidet, daß sie keine Cyclohexylphenylpyrimidinkomponente enthält, folgende Phasenbereiche auf:

X -4 $S_C$ 69 $S_A$ 80 N 92 I

Die Zugabe der erfindungsgemäßen Verbindung führt zu einer Erniedigung des Schmelzpunktes und einer Erhöhung der flüssigkristallinen Phasenbereiche.

Anwendungsbeispiel 7

a) Eine Mischung aus den Komponenten

| | |
|---|---|
| 5-Octyloxy-2-(4-hexyloxy-phenyl)-pyrimidin | 13.0 Mol-% |
| 5-Octyloxy-2-(4-butyloxy-phenyl)-pyrimidin | 14.5 Mol-% |
| 5-Octyloxy-2-(4-decyloxy-phenyl)-pyrimidin | 8 Mol-% |
| 5-Octyloxy-2-(4-octyloxy-phenyl)-pyrimidin | 4.4 Mol-% |
| 5-Octyl-2-(4-dodecyloxy-phenyl)-pyrimidin | 11.8 Mol-% |
| trans-4-Pentyl-cyclohexancarbonsäure-(4'-(5-decylpyrimidin-2-yl))-phenylester | 18.7 Mol-% |
| Heptansäure-(4-(5-octylpyrimidin-2-yl)) phenylester | 19.6 Mol-% |
| 5-(8-Cyclopropyloctyloxy)-2-(4''-trans-propylcyclohexyl-4'-phenyl)-pyrimidin | 10 Mol-% |

zeigt folgende flüssigkristalline Phasenbereiche:

X -8 $S_C$ 76 $S_A$ 80.5 N 101 I

b) Im Vergleich dazu weist die in DE-A 38 31 226 beschriebene flüssigkristalline Mischung, die sich von der obengenannten Mischung nur dadurch unterscheidet, daß sie keine Cyclohexylphenylpyrimidinkomponente enthält, folgende Phasenbereiche auf:

X -7 $S_C$ 73.5 $S_A$ 77 N 96 I

Die Zugabe der erfindungsgemäßen Verbindung führt zu einer Erniedigung des Schmelzpunktes und einer Erhöhung der flüssigkristallinen Phasenbereiche.

Anwendungsbeispiel 8

a) Eine Mischung aus den Komponenten

| | |
|---|---|
| 5-Octyl-2-(4-hexyloxy-phenyl)-pyrimidin | 31,2 Mol-% |
| 5-Octyl-2-(4-octyloxy-phenyl)-pyrimidin | 28,0 Mol-% |
| 5-Octyl-2-(4-decyloxy-phenyl)-pyrimidin | 20,8 Mol-% |
| 5-(8-Cyclopropyloctyloxy)-2-(4''-trans-propylcyclohexyl-4'-phenyl)-pyrimidin | 20 Mol-% |

zeigt folgende flüssigkristalline Phasenbereiche:

X 78 $S_C$ 62 $S_A$ 71 N 87 I

b) Im Vergleich dazu weist die in DE-A 38 31 226 beschriebene flüssigkristalline Mischung, die sich von der obengenannten Mischung nur dadurch unterscheidet, daß sie keine Cyclohexylphenylpyrimidinkomponente enthält, folgende Phasenbereiche auf:

X 17 $S_C$ 51 $S_A$ 60 N 68 I

Die Zugabe der erfindungsgemäßen Verbindung führt zu einer Erniedigung des Schmelzpunktes und einer deutlichen Erhöhung der flüssigkristallinen Phasenbereiche.

c) eine Vergleichsmischung, die Anstelle der erfindungsgemäßen Komponente folgende in DE-A 3915804 beanspruchte Komponente enthält,

trans-4-Pentylcyclohexancarbonsäure-(4''-(5-(9'-Cyclopropylnonyloxy)-pyrimidin-2-yl))phenylester weist folgende flüssigkristallinen Phasen auf:

X 36 $S_C$ 58 N 90 I

Diese Vergleichssubstanz enthält ebenfalls nur zwei aromatische Kerne und unterscheidet sich von der erfindungsgemäßen Substanz in Mischung 7a im wesentlichen durch die Carboxylgruppe. Diese Substanz erhöht in Mischungen auch die flüssigkristalline Phasenbreite ,hat aber den Nachteil, daß sie den Schmelzpunkt der Mischung erhöht und die $S_A$.Phase unterdrückt.

Anwendungsbeispiel 9

a) Eine Mischung aus den Komponenten

| | |
|---|---|
| 5-Octyl-2-(4-hexyloxy-phenyl)-pyrimidin | 25,9 Mol-% |
| 5-Octyl-2-(4-octyloxy-phenyl)-pyrimidin | 23,2 Mol-% |
| 5-Octyl-2-(4-decyloxy-phenyl)-pyrimidin | 17,3 Mol-% |
| 5-(8-Cyclopropyloctyloxy)-2-(4''-trans-propylcyclohexyl-4'-phenyl)-pyrimidin | 13,6 Mol-% |
| 5-[4-(Butyl-dimethylsilyl)butyloxy]-2-(4''-trans -propylcyclohexyl-4'-phenyl)-pyrimidin | 20 Mol-% |

zeigt folgende flüssigkristalline Phasenbereiche:

X -2 $S_C$ 55 N 84 I

b) Im Vergleich dazu weist die in DE-A 38 31 226 beschriebene flüssigkristalline Mischung, die sich von der obengenannten Mischung nur dadurch unterscheidet, daß sie keine Cyclohexylphenylpyrimidinkomponenten enthält, folgende Phasenbereiche auf:

X 17 $S_C$ 51 $S_A$ 60 N 68 I

Die Zugabe der erfindungsgemäßen Verbindungen führt zu einer deutlichen Erniedigung des Schmelzpunktes und einer Erhöhung der flüssigkristallinen Phasenbereiche.

Anwendungsbeispiel 10

a) Eine Mischung aus den Komponenten

| | |
|---|---|
| 5-Octyl-2-(4-hexyloxy-phenyl)-pyrimidin | 25,6 Mol-% |
| 5-Octyl-2-(4-octyloxy-phenyl)-pyrimidin | 23,0 Mol-% |
| 5-Octyl-2-(4-decyloxy-phenyl)-pyrimidin | 17,0 Mol-% |
| 5-(8-Cyclopropyloctyloxy)-2-(4''-trans-propylcyclohexyl-4'-phenyl)-pyrimidin | 14,4 Mol-% |
| 5-(7',7'-Dimethylundecyloxy)-2-(4''-trans -propylcyclohexyl-4'-phenyl)-pyrimidin | 20,0 Mol-% |

zeigt folgende flüssigkristalline Phasenbereiche:

X 13 $S_C$ 67 N 91 I

b) Im Vergleich dazu weist die in DE-A 38 31 226 beschriebene flüssigkristalline Mischung, die sich von der obengenannten Mischung nur dadurch unterscheidet, daß sie keine Cyclohexylphenylpyrimidinkomponenten enthält, folgende Phasenbereiche auf:

X 17 $S_C$ 51 $S_A$ 60 N 68 I

Die Zugabe der erfindungsgemäßen Verbindung führt zu einer deutlichen Erniedigung des Schmelzpunktes und einer Erhöhung der flüssigkristallinen Phasenbereiche.

Anwendungsbeispiel 11

a) Eine Mischung aus den Komponenten

| | |
|---|---|
| 5-Octyl-2-(4-hexyloxy-phenyl)-pyrimidin | 27,3 Mol-% |
| 5-Octyl-2-(4-decyloxy-phenyl)-pyrimidin | 18,2 Mol-% |
| 5-Decyl-2-(4-hexyloxy-phenyl)-pyrimidin | 17,7 Mol-% |
| 5-Octyl-2-(4-heptylcarbonyloxy-phenyl) -pyrimidin | 16,8 Mol-% |
| 5-(8-Cyclopropyloctyloxy)-2-(4"-trans-pentylcyclohexyl-4'-phenyl)-pyrimidin | 20 Mol-% |

zeigt folgende flüssigkristalline Phasenbereiche:

X -1 $S_C$ 61 $S_A$ 72 N 84 I

b) Im Vergleich dazu weist die flüssigkristalline Mischung, die sich von der obengenannten Mischung nur dadurch unterscheidet, daß sie keine Cyclohexylphenylpyrimidinkomponenten enthält, folgende Phasenbereiche auf:

X 8 $S_C$ 48 $S_A$ 61 N 65 I

Die Zugabe der erfindungsgemäßen Verbindung führt zu einer deutlichen Erniedigung des Schmelzpunktes und einer Erhöhung der flüssigkristallinen Phasenbereiche. Dieses Beispiel belegt, daß die erfindungsgemäßen Substanzen insbesondere bei Mischungen geeignet sind, die überwiegend aus Alkyl-(Alkyloxy-phenyl)-pyrimidinen bestehen.

Anwendungsbeispiel 12

a) Eine Mischung aus den Komponenten

| | |
|---|---|
| 5-Octyl-2-(4-hexyloxy-phenyl)-pyrimidin | 24,1 Mol-% |
| 5-Octyl-2-(4-decyloxy-phenyl)-pyrimidin | 16,1 Mol-% |
| 5-Decyl-2-(4-hexyloxy-phenyl)-pyrimidin | 15,6 Mol-% |
| 5-Octyl-2-(4-heptylcarbonyloxy-phenyl) -pyrimidin | 12,2 Mol-% |
| 5-(8-Cyclopropyloctyloxy)-2-(4''-trans-pentylcyclohexyl-4'-phenyl)-pyrimidin | 16,0 Mol-% |
| 5-(7'-7'-Dimethylundecyloxy)-2-(4''-trans-propylcyclohexyl-4'-phenyl)-pyrimidin | 16,0 Mol-% |

zeigt folgende flüssigkristalline Phasenbereiche:

X -6 $S_C$ 68 $S_A$ 72 N 89 I

b) Im Vergleich dazu weist die flüssigkristalline Mischung, die sich von der obengenannten Mischung nur dadurch unterscheidet, daß sie keine Cyclohexylphenylpyrimidinkomponenten enthält, folgende Phasenbereiche auf.

X 8 $S_C$ 48 $S_A$ 61 N 65 I

Die Zugabe der erfindungsgemäßen Verbindungen führt zu einer deutlichen Erniedrigung des Schmelzpunktes und einer Erhöhung der flüssigkristallinen Phasenbereiche. Dieses Beispiel belegt, daß die erfindungsgemäßen Substanzen insbesondere bei Mischungen geeignet sind, die überwiegend aus 5-Alkyloxy-2-(4-alkyloxyphenyl)-pyrimidinen, 5-Alkyl-2-(4-alkyloxyphenyl)-pyrimidinen, bzw. entsprechenden Phenylpyridinen bestehen und die für den Anwender zu schmale flüssigkristalline Phasen aufweisen.

Anwendungsbeispiel 13

a) Eine Mischung besteht aus den Komponenten

| | |
|---|---|
| 5-Octyl-2-(4-hexyloxy-phenyl)-pyrimidin | 34,6 Mol-% |
| 5-Octyl-2-(4-decyloxy-phenyl)-pyrimidin | 23,0 Mol-% |
| 5-Decyl-2-(4-hexyloxy-phenyl)-pyrimidin | 22,4 Mol-% |
| 5-(8-Cyclopropyloctyloxy)-2-(4''-trans-pentylcyclohexyl-4'-phenyl)-pyrimidin | 20,0 Mol-% |

und zeigt folgende flüssigkristalline Phasenbereiche:

X 8.5 $S_C$ 63 $S_A$ 75 N 87 I

b) Im Vergleich dazu weist die in DE-A 38 31 226 beschriebene flüssigkristalline Mischung, die sich von der der obengenannten Mischung nur dadurch unterscheidet, daß sie keine Cyclohexylphenylpyrimidinkomponenten enthält, folgende Phasenbereiche auf:

X 12 $S_C$ 47 $S_A$ 63 N 67 I

Die Zugabe der erfindungsgemäßen Verbindung führt zu einer Erniedigung des Schmelzpunktes und einer Erhöhung der flüssigkristallinen Phasenbereiche.

Anwendungsbeispiel 14

Eine ferroelektrische Mischung besteht aus der

| | |
|---|---|
| Beispiel-Mischung 12 zu | 94,73 Mol-% |
| (2S,3S)-2-[4-(5-Octyl-pyrimidin-2-yl)-phenoxy]-methyl-3-butyl-oxiran | 1,94 Mol-% |
| (S)-4-(2-Octyloxypyrimidin-5-yl)-phenyl-[spiro-(1,3-dioxolan-2,1'-cyclohexan)-4-yl] methyl-ether | 1,14 Mol-% |
| (2R,3R)-3-Propyl-oxiran-2-carbonsäure-[4-(2-octyloxy-pyrimidin-5-yl)-phenyl]-ester | 2,19 Mol-% |

und weist folgende Phasen auf:

X -7 $S_C$ 67 $S_A$ 71 N 87 I

Diese Mischung weist bei 25°C eine Polarisation von 10 $nCcm^2$ auf und schaltet in einer 2 µm di cken Schicht bei einer Feldstärke von 10 $V\mu m^{-1}$ mit einer Schaltzeit von 113 µs.

In den nachfolgenden Tabellen werden die Temperaturen für Phasenumwandlungen in °C angegeben. Monotrope Phasenumwandlungen sind in Klammern angegeben.

Tabelle 1

Beispielsubstanzen der allgemeinen Formel (II)

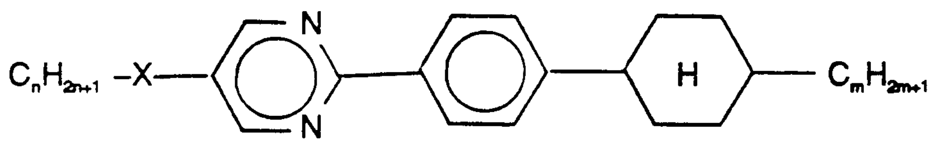 (II)

5-Alkyl(oxy)-2-(4"-alkyl-trans-cyclohexan-4'-phenyl)-pyrimidine

| Beispiel | | | | Phasenumwandlungstemperaturen* | | | | | |
|---|---|---|---|---|---|---|---|---|---|
| Nr. | n | x | m | X | $S_x$ | $S_c$ | $S_A$ | N | I |
| 1 | 8 | 0 | 5 | 65 | 83 | 119 | - | 181 | |
| 2 | 8 | - | 5 | 74 | - | 88 | 103 | 158 | |

Tabelle 2

Beispielsubstanzen der allgemeinen Formel (III)

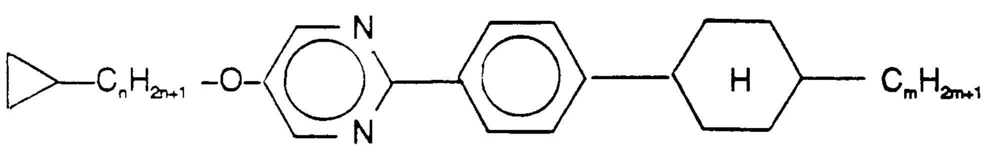 (III)

5-(Cyclopropylalkoxy)-2-(4"-alkyl-trans-cyclohexan-4'-phenyl)-pyrimidin

| Beispiel | | | Phasenumwandlungstemperaturen* | | | | | |
|---|---|---|---|---|---|---|---|---|
| Nr. | n | m | X | $S_x$ | $S_c$ | $S_A$ | N | I |
| 3 | 4 | 5 | 81 | (75) | - | - | 180 | |
| 4 | 6 | 5 | 63 | 93 | 100 | - | 173 | |
| 5 | 8 | 5 | 75 | - | 122 | 135,5 | 264 | |
| 6 | 6 | 3 | 80 | (70) | 84 | - | 174 | |
| 7 | 8 | 3 | 86 | - | 111 | 123 | 165 | |

Anhand der Beispiele in Tabelle 2 erkennt man, daß die Komponenten mit der Cyclopropylhexyloxygruppe bevorzugt sind, da sie in der homologen Reihe den niedrigsten Schmelzpunkt aufweisen. Die Verbindungen mit der Cyclopropyloctyloxygruppe sind besonders bevorzugt, da sie die gewünschen flüssigkristalline Phasen und keine höhergeordnete flüssigkristalline Phase enthalten. Der Vergleich mit Beispielverbindung 1 aus Tabelle 1 mit der Verbindung aus Beispiel 5 aus Tabelle 2 zeigt, daß die

Verbindungen, die anstelle einer $C_2H_5$-Gruppe eine Cyclopropylgruppe enthalten, einen niedrigeren Schmelzpunkt aufweisen als die geradkettige Substanz (Beispiel 1).

## Tabelle 3

## Beispielsubstanzen der allgemeinen Formel (IV)

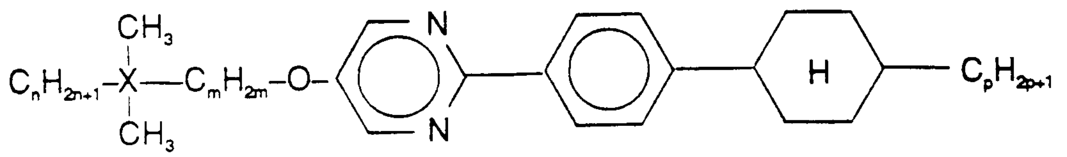

(IV)

5-(Alkyl-dimethyl-(silyl)-alkyloxy)-2-(4"-alkyl-trans-cyclohexan-4'-phenyl)-pyrimidin

| Beispiel Nr. | n | x | m | p | Phasenumwandlungstemperaturen* $S_X$ | $S_C$ | $S_A$ | N | I |
|---|---|---|---|---|---|---|---|---|---|
| 8 | 4 | Si | 4 | 5 | 52 | (48) | - | 87 | |
| 9 | 4 | Si | 4 | 3 | 46 | 60 | - | 100 | |
| 10 | 4 | C | 6 | 3 | 48 | - | 85 | 117 | |

Anhand der Verbindungen in Tabelle 3 erkennt man, daß unter den Butyldimethylverzweigten Verbindungen die Verbindungen mit einem Hexyloxy-Spacer bevorzugt sind, da sie anstelle einer höhergeordneten Phase eine $S_C$-Phase und einen relativ hohen Klärpunkt aufweisen.

Der Vergleich der Beispielverbindung 8 mit der Beispielverbindung 1 aus Tabelle 1 zeigt, daß die Einführung einer Dimethyl-verzweigten Gruppe zu einer Erniedrigung des Schmelzpunktes von 31 ° C führt.

Außerdem weisen diese dreikernigen Verbindungen auch in Vergleich zu anderen Dimethyl-verzweigten dreikernigen Verbindungen sehr niedrige Schmelzpunkte auf. So weist die in DE-A 3 827 600 beschriebene Verbindung (siehe Beispiel 57, 5-trans-Pentylcyclohexancarbonsäure-[4-(5-(4-butyldimethylsilyl)-butloxypyrimidin-2-yl)phenyl]-ester), die sich von der erfindungsgemäßen Beispielverbindung 8 nur dadurch unterscheidet, daß sie noch eine Carboxylgruppe mehr enthält, einen um 30 ° C höheren Schmelzpunkt auf.

**Patentansprüche**

**1.** Cyclohexylphenylpyrimidin der allgemeinen Formel (I)

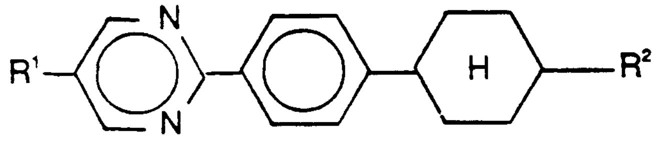

(I)

in der bedeuten:

$R^1$ geradkettiges oder verzweigtes, chirales oder achirales Alkyl oder Alkenyl mit 2 bis 16 C-Atomen, wobei eine oder zwei nicht benachbarte $-CH_2$-Gruppen durch -O-,-S-,-CO-, -COO-, -OCO-, $-Si(CH_3)_2$- oder $-C(CH_3)_2$- ersetzt sein können, und wobei auch ein oder mehrere H-Atome des Alkyl- bzw. Alkenylrestes durch F-Atome ersetzt sein können, und wobei die endständige $CH_3$-Gruppe des Alkyls auch durch

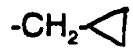

ersetzt sein kann,

$R^2$ geradkettiges oder verzweigtes, chirales oder achirales Alkyl mit 1 bis 10 C-Atomen.

**2.** Cyclohexylphenylpyrimidin gemäß Anspruch 1, dadurch gekennzeichnet, daß in Formel (I),
$R^1$ ein geradkettiges oder verzweigtes, chirales oder achirales Alkyl mit 2 bis 16 C-Atomen ist, bei dem eine $-CH_2$-Gruppe durch $-C(CH_3)_2$- oder - $Si(CH_3)_2$ ersetzt ist und bei dem eine dazu nicht benachbarte $-CH_2$-Gruppe durch -O-, -S-, -COO- oder -OCO- ersetzt sein kann.

**3.** Cyclohexylphenylpyrimidin gemäß Anspruch 1, dadurch gekennzeichnet, daß in Formel I
$R^1$ ein geradkettiges oder verzweigtes, chirales oder achirales Alkyl mit 2 bis 16 C-Atomen ist, bei dem die endständige $CH_3$-Gruppe durch

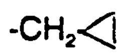

ersetzt ist, und bei dem eine $-CH_2$-Gruppe durch -O-, -S-, -COO- oder -O-CO- ersetzt sein kann.

**4.** Cyclohexylphenylpyrimidin gemäß Anspruch 1, dadurch gekennzeichnet, daß in Formel (I)
$R^1$ ein geradkettiges Alkyl mit 4 bis 12 C-Atomen ist, wobei eine $-CH_2$-Gruppe durch -O- oder -S- ersetzt sein kann.

**5.** Cyclohexylphenylpyrimidin gemäß Anspruch 1, dadurch gekennzeichnet, daß in Formel (I)
$R^1$ ein geradkettiges Alkyl mit 4 bis 12 C-Atomen bedeutet, bei dem eine - $CH_2$-Gruppe durch $-C(CH_3)_2$- ersetzt ist und eine weitere, dazu nicht benachbarte, durch -O- ersetzt sein kann.

**6.** Cyclohexylphenylpyrimidin gemäß Anspruch 1, dadurch gekennzeichnet, daß in Formel (I)
$R^1$ ein geradkettiges Alkyl mit 4 bis 12 C-Atomen bedeutet, bei dem die endständige $CH_3$-Gruppe durch

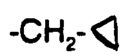

ersetzt ist.

**7.** Cyclohexylphenylpyrimidin gemäß Anspruch 1, dadurch gekennzeichnet, daß in Formel (I)
$R^2$ geradkettiges Alkyl mit 2 bis 8 C-Atomen bedeutet.

**8.** Verwendung eines Cyclohexylphenylpyrimidins gemäß einem der Ansprüche 1 bis 7 als Komponente in ferroelektrischen Flüssigkristallmischungen.

**9.** Flüssigkristalline Mischung bestehend aus mindestens zwei Komponenten, dadurch gekennzeichnet, daß sie als eine Komponente ein Cyclohexylphenylpyrimidin gemäß Anspruch 1 enthält.

**10.** Ferroelektrische Schalt- und Anzeigevorrichtung enthaltend Trägerplatten, Elektroden, mindestens einen Polarisator, gegebenenfalls Orientierungsschichten sowie ein flüssigkristallines Medium, dadurch gekennzeichnet, daß das flüssigkristalline Medium eine ferroelektrische Flüssigkristallmischung gemäß Anspruch 9 ist.

**Patentsprüche für folgende Vertragsstaaten: ES, GR**

**1.** Verwendung dieses Cyclohexylphenylpyrimidins der Formel I

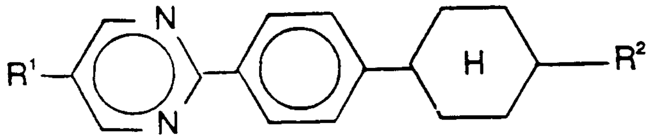

(I)

in der bedeuten:

$R^1$ geradkettiges oder verzweigtes, chirales oder achirales Alkyl oder Alkenyl mit 2 bis 16 C-Atomen, wobei eine oder zwei nicht benachbarte $-CH_2-$ Gruppen durch -O-, -S-, -CO-, -COO-, -OCO-, $-Si(CH_3)_2-$ oder $-C(CH_3)_2-$ ersetzt sein können, und wobei auch ein oder mehrere H-Atome des Alkyl- bzw. Alkenylrestes durch F-Atome ersetzt sein können, und wobei die endständige $CH_3$-Gruppe des Alkyls auch durch

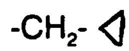

ersetzt sein kann,

$R^2$ geradkettiges oder verzweigtes, chirales oder achirales Alkyl mit 1 bis 10 C-Atomen,

als Komponente in ferroelektrischen Flüssigkristallmischungen.

**2.** Ferroelektrische Schalt- und Anzeigevorrichtung enthaltend Trägerplatten, Elektroden, mindestens einen Polarisator, gegebenenfalls Orientierungsschichten sowie ein flüssigkristallines Medium, dadurch gekennzeichnet, daß das flüssigkristalline Medium eine Verbindung der Formel I ist

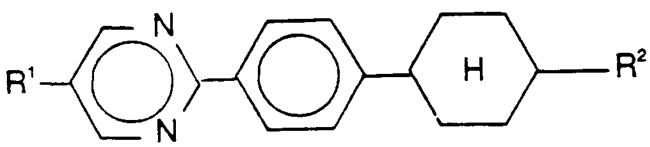

(I)

in der bedeuten:

$R^1$ geradkettiges oder verzweigtes, chirales oder achirales Alkyl oder Alkenyl mit 2 bis 16 C-Atomen, wobei eine oder zwei nicht benachbarte $-CH_2-$ Gruppen durch -O-, -S-, -CO-, -COO-, -OCO-, $-Si(CH_3)_2-$ oder $-C(CH_3)_2-$ ersetzt sein können, und wobei auch ein oder mehrere H-Atome des Alkyl- bzw. Alkenylrestes durch F-Atome ersetzt sein können, und wobei die endständige $CH_3$-Gruppe des Alkyls auch durch

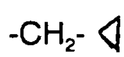

ersetzt sein kann,

$R^2$ geradkettiges oder verzweigtes, chirales oder achirales Alkyl mit 1 bis 10 C-Atomen.